# EUROPÄISCHE PATENTSCHRIFT

(11) **EP 3 011 224 B1**
(45) Veröffentlichungstag und Bekanntmachung des Hinweises auf die Patenterteilung: **21.06.2017**
(21) Anmeldenummer: 14725623.4
(22) Anmeldetag: 25.04.2014
(51) Int. Cl.: F16N 29/00, F16N 29/04, G01N 21/00, G01N 21/01, G01N 21/15, G01N 21/17, G01N 33/28

(54) **SCHMIERSTOFFSENSOR**
LUBRICANT SENSOR
SENSEUR D'HUILE

(30) Priorität: 19.06.2013 DE 102013211486
(43) Veröffentlichungstag der Anmeldung: 27.04.2016
(73) Patentinhaber: Schaeffler Technologies AG & Co. KG, 91074 Herzogenaurach (DE)
(72) Erfinder: NEUSCHAEFER-RUBE, Stephan, 91074 Herzogenaurach (DE)
(86) Internationale Anmeldenummer: PCT/DE2014/200182
(87) Internationale Veröffentlichungsnummer: WO 2014/202066

(56) Entgegenhaltungen:
- EP-A1- 0 635 714
- GB-A- 2 332 755
- GB-A- 2 408 798
- JP-A- S 554 579
- US-A- 3 603 952

## Beschreibung

### Gebiet der Erfindung

Die Erfindung betrifft einen Schmierstoffsensor, umfassend ein Gehäuse, eine im Gehäuse angeordnete IR-Lichtquelle, einen im Gehäuse angeordneten ersten IR-Detektor und eine in eine Öffnung des Gehäuses eingesetzte Scheibe, wobei die Scheibe im IR-Spektralbereich durchlässig ist. Die Erfindung betrifft weiter ein Wälzlager mit einem derartigen Schmierstoffsensor.

### Hintergrund der Erfindung

Üblicherweise wird ein Wälzlager mit einem Schmierstoff, z.B. Fett, geschmiert, um z.B. Reibungsverluste zu minimieren. Durch den Betrieb eines Wälzlagers verändern sich jedoch die Materialeigenschaften des eingesetzten Schmierstoffes, beispielsweise durch hohe Betriebstemperaturen oder durch den Abrieb kleiner Metallpartikel an den Kontaktflächen im Wälzlager, so dass seine Schmierfähigkeit mit zunehmender Betriebsdauer nachlassen kann. Um Schäden am Wälzlager durch eine mangelnde Schmierung vorzubeugen, sollte die Beschaffenheit des Schmierstoffes bedarfsweise überwacht werden. Insbesondere bei einem schwer zugängigen Wälzlager, wie es z.B. in einer Windkraftanlage Verwendung findet, ist es oft wünschenswert, den Schmierstoff direkt im Betrieb überwachen zu können, da aus Effizienzgründen die Entnahme einer Probe zur Analyse nur schwer realisierbar ist. Hierzu kann in einem Wälzlager ein Sensor eingebracht sein, welcher dazu ausgelegt ist, die Streuung von infrarotem Licht verschiedener Wellenlängen durch den Schmierstoff zu messen. Hierbei wird unter infrarotem Licht generell der Spektralbereich von Wellenlängen zwischen 700 nm und 1 mm verstanden. Nach der Signalverarbeitung der Messwerte kann man Informationen über Wassergehalt, Trübung und Verschleiß des Schmierstoffes gewinnen.

Ein solcher Sensor umfasst üblicherweise eine Anzahl von LEDs als Infrarot (IR)-Lichtquelle und eine Anzahl von Infrarot-Detektoren. Zum Schutz vor Schmierstoffeinwirkung sind die genannten Komponenten in ein Gehäuse eingebracht, welches an einer Seite von einer IR-durchlässigen Scheibe abgeschlossen ist, die aus einem möglichst kratzfesten und hitzebeständigen Glas oder Kristall wie z.B. Saphirglas gefertigt ist. Geeignete Materialien für die Scheibe weisen hierbei einen vergleichsweise hohen Brechungsindex auf. Durch die unterschiedlichen Brechungsindizes der Scheibe und der Luft im Sensorinnenraum entstehen an der inneren Grenzfläche der Scheibe selbst bei senkrechtem Lichteinfall Reflexionen. Dies führt zum einen dazu, dass im Sensorinnenraum erzeugtes Licht teilweise sofort zurückreflektiert wird, wodurch eine hohe Offset-Lichtleistung detektiert wird. Dies bedeutet, dass unabhängig der von außen durch die Scheibe in den Sensor zurückgestreuten Lichtleistung an einem Detektor dauerhaft teilreflektiertes Licht einfällt, was zu einem konstanten Offset des Nullpunkts für die von außen durch die Scheibe einfallende Leistung am Detektor führt. Zum anderen kann am Schmierstoff gestreutes Licht, welches durch die Scheibe in den Sensor eintritt, aufgrund der Reflexion nur teilweise detektiert werden, was die Empfindlichkeit des Sensors gegenüber Zustandsänderungen des Schmierstoffes verringert. Eine höhere Offset-Lichtleistung sowie verringerte Sensorempfindlichkeit durch Reflexionen können auch an der äußeren Grenzfläche der Scheibe auftreten, insbesondere, wenn der Schmierstoff nicht dauerhaft in direktem Kontakt mit der Scheibe steht.

Die GB 2 408 798 A offenbart einen Schmierstoffsensor nach dem Oberbegriff des Anspruchs 1.

### Aufgabe der Erfindung

Der Erfindung liegt die Aufgabe zugrunde, einen optischen Schmierstoffsensor anzugeben, welcher eine möglichst geringe Offset-Leistung detektiert und möglichst empfindlich für am Schmierstoff gestreutes Licht ist. Des Weiteren soll eine vorteilhafte Anwendung für einen derartigen Schmierstoffsensor angegeben werden.

### Zusammenfassung der Erfindung

Die Aufgabe wird für einen Schmierstoffsensor, umfassend ein Gehäuse, eine im Gehäuse angeordnete IR-Lichtquelle, einen im Gehäuse angeordneten ersten IR-Detektor, und eine in eine Öffnung des Gehäuses eingesetzte Scheibe, wobei die Scheibe im IR-Spektralbereich durchlässig ist, erfindungsgemäß dadurch gelöst, dass die Scheibe zum Innenraum des Gehäuses hin zumindest für den Spektralbereich der IR-Lichtquelle antireflexbeschichtet ist.

Der Erfindung liegt dabei in einem ersten Schritt die Überlegung zugrunde, dass die Scheibe durch ihre Materialeigenschaften dazu ausgelegt ist, den im Gehäuse angeordneten Komponenten einen möglichst wirksamen Schutz vor Belastungen, welche außerhalb des Sensors auftreten können, bieten soll, und dass daher die Beschaffenheit der Scheibe an der Außenseite nicht verändert werden sollte. Hierbei bezieht sich die Innenseite der Scheibe auf den Innenraum des Gehäuses.

In einem zweiten Schritt erkennt die Erfindung, dass eine erhöhte Offset-Lichtleistung und eine verringerte Empfindlichkeit durch die Reflexion an den Grenzflächen entstehen, und somit durch eine Antireflexbeschichtung an der Fläche zum Innenraum des Gehäuses hin eine verbesserte Auflösung des Schmierfettsensors ermöglicht wird. Der Reflexionskoeffizient R ist für jede senkrechte Grenzflächendurchdringung durch R = (n-1)²/(n+1)² gegeben, wobei n den Brechungsindex der Scheibe bezeichnet. Bei für die Scheibe geeigneten Materialien, welche im IR-durchlässig sind, kann der Brechungsindex Werte von 1.77 (Saphirglas) oder über 2.6 (Chalkogenid-Gläser) betragen. Dies führt zu einer Reflexion von 8% bzw. sogar über 20%. Von der IR-Lichtquelle emittiertes Licht wird zum durch den Reflexionskoeffizienten gegebenen Prozentsatz an der inneren Grenzfläche der Scheibe zum ersten Infrarotdetektor reflektiert und dort als Offset-Lichtleistung detektiert, während am Schmierstoff gestreutes Licht zum gegebenen Prozentsatz an der selben Fläche vom Sensor weg nach außen reflektiert wird und somit nicht detektiert wird. Eine Antireflexbeschichtung an der Innenseite der Scheibe kann beide Effekte auf unter 1% reduzieren.

Etwaige optische Verluste durch Reflexionen an der Außenseite der Scheibe halten sich in tolerablen Grenzen, solange die Scheibe an der Außenseite in direktem Kontakt mit einem Schmierstoff steht, da dieser einen höheren Brechungsindex als Luft aufweist. Ein typischer Wert für den Brechungsindex eines Schmierstoffes ist ca. 1.47. In Anbetracht der Tatsache, dass die Materialeigenschaften der Scheibe an der Außenseite nicht verändert werden sollten, kann es somit ausreichend sein, den Schmierstoffsensor so zu positionieren, dass die Scheibe im Betrieb möglichst in direktem Kontakt mit dem Schmierstoff ist.

Vorteilhafterweise ist die Scheibe aus Saphirglas gefertigt. Saphirglas ist durch seine hohe Härte besonders kratzfest, zudem weist es im infraroten Spektralbereich einen für vergleichbare Materialien niedrigen Brechungsindex von 1.77 auf. Steht die Scheibe an ihrer Außenseite in direktem Kontakt mit dem Schmierstoff, können zudem Reflexionen dort praktisch als vernachlässigbar angesehen werden. In diesem Fall wird im Wesentlichen kein IR-Licht von der IR-Lichtquelle von der äußeren Grenzfläche der Scheibe zum ersten IR-Detektor reflektiert, und am Schmierstoff gestreutes IR-Licht, welches senkrecht auf die Scheibe auftrifft, wird im Wesentlichen vollständig in den Innenraum des Sensors durchgelassen.

Die IR-Lichtquelle ist hierbei bevorzugt als Licht-emittierende Diode (LED) ausgebildet. Dies hat den Vorteil einer kompakten Bauweise und eines geringen Stromverbrauchs.

Günstigerweise emittiert die IR-Lichtquelle Licht im nahen und/oder mittleren Infrarotbereich. Der nahe Infrarotbereich bezeichnet hierbei den Spektralbereich mit Wellenlängen von 700 nm bis 3 µm, der mittlere Infrarotbereich umfasst Wellenlängen von 3 µm bis 50 µm. Im Grenzbereich von nahem und mittlerem Infrarotbereich befinden sich Spektrallinien von Molekülschwingungen, insbesondere von Streckschwingungen, welche in Kohlenwasserstoffen auftreten. Durch Ermitteln derartiger Spektrallinien im Absorptionsspektrum des Schmierstoffes lassen sich wichtige Informationen über dessen chemische Eigenschaften in Erfahrung bringen.

Im Gehäuse ist ein zweiter IR-Detektor angeordnet. Der erste IR-Detektor ist dabei bevorzugt als ein Messdetektor für am Schmierstoff gestreutes In-Licht vorgesehen, während der zweite IR-Detektor bevorzugt als ein Referenzdetektor für von der IR-Lichtquelle emittiertes Licht vorgesehen ist. Somit lässt sich der bezüglich emittierten IR-Lichtes zurückgestreute Anteil ermitteln, was eine genauere Kalibrierung ermöglicht. Hierbei ist durch bauliche Anordnung der zweite IR-Detektor im Gehäuse vor dem am Schmierstoff gestreuten, einfallenden Licht geschützt, und der erste IR-Detektor vor direkter Bestrahlung durch die IR-Lichtquelle geschützt. Insbesondere kann dies dadurch erreicht werden, dass der erste Detektor und der zweite Detektor bezüglich durch die Scheibe einfallender Strahlen im Wesentlichen hintereinander angeordnet sind, so dass durch die Scheibe eintretendes IR-Licht nur auf den ersten IR-Detektor trifft. In diesem Fall kann mittels weiterer im Gehäuse angeordneter, verspiegelter Elemente Licht von der IR-Lichtquelle zum zweiten IR-Detektor geleitet werden.

Günstigerweise ist im Gehäuse eine weitere IR-Lichtquelle angeordnet, wobei die Scheibe im Spektralbereich jeder IR-Lichtquelle durchlässig ist, und wobei die Scheibe zum Innenraum des Gehäuses hin für den Spektralbereich jeder IR-Lichtquelle antireflexbeschichtet ist. Bevorzugt emittieren hierbei die beiden IR-Lichtquellen im Betrieb des Schmierstoffsensors in unterschiedlichen Wellenlängenbereichen. Dies erlaubt eine breite spektrale Auflösung der Streuung von IR-Licht am Schmierstoff.

Als vorteilhaft erweist es sich weiter, wenn die oder jede IR-Lichtquelle und der oder jeder IR-Detektor auf einer Platine angeordnet sind. Durch die Anordnung der genannten Komponenten auf einer Platine wird eine besonders kompakte Bauweise und ein vereinfachter Einbau ermöglicht.

Bevorzugt ist hierbei die Platine flexibel ausgebildet. Somit ist eine gewünschte kompakte Bauweise besonders einfach zu erreichen. Insbesondere lässt sich über eine flexible Platine durch Faltung, ggf. mittels weiterer verspiegelter und/oder IR-transparenter Elemente ein gewünschter Strahlengang vor dem Einbau aller Komponenten in das Gehäuse einstellen, was die Konstruktion vereinfacht.

In einer weiter vorteilhaften Ausführung der Erfindung ist im Gehäuse eine Elektronik eingebracht, welche mit dem oder jedem IR-Detektor verbunden ist. Die Elektronik kann bevorzugt dazu ausgelegt sein, Signale des oder jeden IR-Detektors zu digitalisieren und zu verstärken. Durch eine Digitalisierung und/oder Verstärkung von IR-Detektorsignalen noch im Gehäuse wird die Signalverarbeitung unempfindlicher gegen äußere Einflüsse wie Vibrationen oder Temperaturänderungen in der Umgebung des Sensors.

Die zweitgenannte Aufgabe wird erfindungsgemäß durch ein Wälzlager mit einem vorbeschriebenen Schmierstoffsensor gelöst. Die für den Schmierstoffsensor und dessen Weiterbildungen angegebenen Vorteile können hierbei sinngemäß auf das Wälzlager übertragen werden.

### Kurze Beschreibung der Zeichnungen

Ein Ausführungsbeispiel der Erfindung wird anhand einer Zeichnung näher erläutert. Dabei zeigen:
- Fig. 1:: schematisch einen Schmierstoffsensor mit antireflexbeschichteter Scheibe in Querschnittdarstellung, und
- Fig. 2:: schematisch ein Wälzlager mit einem Schmierstoffsensor in der axialen Draufsicht.

### Detaillierte Beschreibung der Zeichnungen

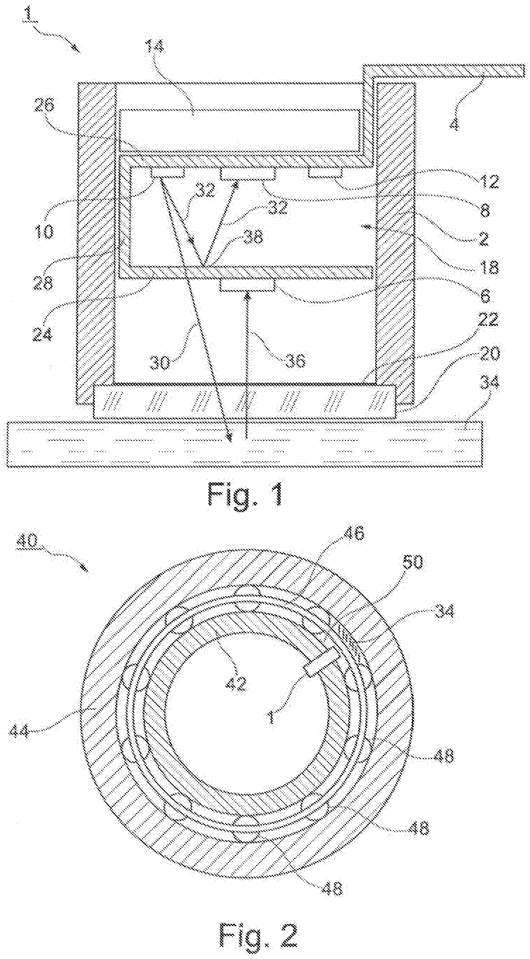

In Fig. 1 ist schematisch ein Schmierstoffsensor 1 dargestellt. In einem Gehäuse 2 ist eine flexible Platine 4 eingebracht, auf welcher ein erster IR-Detektor 6, ein zweiter IR-Detektor 8 und zwei LEDs 10, 12 angeordnet sind. Der erste IR-Detektor 6 ist hierbei als ein Messdetektor, der zweite IR-Detektor als ein Referenzdetektor ausgebildet. An der Platine 4 ist weiter eine Elektronik 14 angeordnet. Der Innenraum 18 des Gehäuses 2 ist in einer Richtung von einer Scheibe 20 aus Saphirglas begrenzt, welche mit einer Antireflexschicht 22 versehen. Die flexible Platine 4 ist in diesem Ausführungsbeispiel derart gefaltet, dass im Innenraum 18 parallel zur Scheibe 20 zwei Platinenflächen 24, 26 einliegen, welche durch einen Steg 28 miteinander verbunden sind. Auf der ersten Platinenfläche 24 ist, zur Scheibe 20 hin gewandt, der erste IR-Detektor 6 angebracht, während auf der bezüglich der Scheibe 20 dahinter liegenden zweiten Platinenfläche 26, ebenfalls zur Scheibe 20 hin gewandt, der zweite IR-Detektor 8 und die beiden LEDs 10, 12 angeordnet sind. Auf der anderen Seite der Platinenfläche 26 ist die Elektronik 14 angebracht. Die LED 10 emittiert im Betrieb des Schmierstoffsensors 1 IR-Licht 30, 32, wobei der Strahlengang 30 durch eine nicht näher dargestellte Öffnung in der Platinenfläche 24 zur Scheibe 20 gelangt. Aufgrund der Antireflexbeschichtung 22 der Saphirglas-Scheibe 20 verlässt das IR-Licht 30 den Schmierstoffsensor 1 und trifft vor der Scheibe 20 auf einen Schmierstoff 34. Ein Teil 36 des IR-Lichtes 30 wird vom Schmierstoff 34 durch die Scheibe 20 zurück in den Schmierstoffsensor 1 gestreut und trifft dort auf den ersten IR-Detektor 6. Der Strahlengang 32 wird an einem Spiegelelement 38 auf der Rückseite der Platinenfläche 24 direkt zum zweiten IR-Detektor 8 gespiegelt. Für die LED 12, welche andere Wellenlängen als die LED 10 emittiert, gelten ähnliche Strahlengänge wie 30, 32 für die LED 10. Der erste IR-Detektor 6 und der zweite IR-Detektor 8 sind mit der Elektronik 14 verbunden, welche jeweils die Detektionssignale digitalisiert und verstärkt. Aus den Signalen des ersten IR-Detektors 6 und des zweiten IR-Detektors 8 können in einer nicht näher dargestellten Auswerteeinheit Informationen über die Beschaffenheit des Schmierstoffes 34 gewonnen werden.

In Fig. 2 ist in schematischer Draufsicht ein Wälzlager 40 mit einem Schmierstoffsensor 1 dargestellt. Das Wälzlager umfasst einen Innenring 42, einen Außenring 44 und einen Wälzkörperkäfig 46 mit darin angeordneten Wälzkörpern 48. In diesem Ausführungsbeispiel ist der Außenring 44 des Wälzlagers 40 als drehend und der Innenring 42 als fest stehend eingesetzt. An einer Stirnfläche des Innenrings 42 ragt schräg, also in radial-axialer Richtung, der Schmierstoffsensor 1 in den Zwischenraum 50 zwischen Innenring 42 und Außenring 44, ohne dabei jedoch die Bewegung der 48 Wälzkörper zu beeinträchtigen. Während der Rotation des Außenrings 44 kann so der Schmierstoffsensor 1 einen Schmierstoff 34 untersuchen und Messdaten an eine nicht näher dargestellte Auswerteinheit senden.

### Bezugszeichenliste

- 1: Schmierstoffsensor
- 2: Gehäuse
- 4: flexible Platine
- 6: erster In-Detektor
- 8: zweiter IR-Detektor
- 10: erste LED als IR-Lichtquelle
- 12: zweite LED als IR-Lichtquelle
- 14: Elektronik
- 18: Innenraum des Schmierstoffsensors
- 20: Scheibe
- 22: Antireflexbeschichtung
- 24: erste Platinenfläche
- 26: zweite Platinenfläche
- 28: Steg an der Platine
- 30: IR-Licht (aus dem Schmierstoffsensor emittiert)
- 32: IR-Licht (zum IR-Referenzdetektor geleitet)
- 34: Schmierstoff
- 36: IR-Licht (am Schmierstoff gestreut)
- 38: Spiegelelement
- 40: Wälzlager
- 42: Innenring
- 44: Außenring
- 46: Wälzkörperkäfig
- 48: Wälzkörper
- 50: Zwischenraum zwischen Innen- und Außenring

## Patentansprüche

1. Schmierstoffsensor (1), umfassend ein Gehäuse (2), eine im Gehäuse (2) angeordnete IR-Lichtquelle (10), einen im Gehäuse (2) angeordneten ersten IR-Detektor (6), und eine in eine Öffnung des Gehäuses eingesetzte Scheibe (20), wobei die Scheibe (20) im Spektralbereich der IR-Lichtquelle (10) durchlässig ist, **dadurch gekennzeichnet, dass** die Scheibe (20) zum Innenraum (18) des Gehäuses hin zumindest für den Spektralbereich der IR-Lichtquelle (10) antireflexbeschichtet ist, und dass im Gehäuse (2) ein zweiter IR-Detektor (8) angeordnet ist, wobei der erste IR-Detektor (6) derart angeordnet ist, dass er vor direkter Bestrahlung durch die IR-Lichtquelle (10) geschützt ist, und wobei der zweite IR-Detektor (8) derart angeordnet ist, dass er vor dem am Schmierstoff gestreuten, einfallenden Licht geschützt ist.

2. Schmierstoffsensor (1) nach Anspruch 1, **dadurch gekennzeichnet, dass** die Scheibe (20) aus Saphirglas gefertigt ist.

3. Schmierstoffsensor (1) nach Anspruch 1 oder Anspruch 2, **dadurch gekennzeichnet, dass** die IR-Lichtquelle (10) Licht (30, 32) im nahen und/oder mittleren Infrarotbereich emittiert.

4. Schmierstoffsensor (1) nach einem der vorhergehenden Ansprüche, **dadurch gekennzeichnet, dass** im Gehäuse (2) eine weitere IR-Lichtquelle (12) angeordnet ist, wobei die Scheibe (20) im Spektralbereich jeder IR-Lichtquelle (10, 12) durchlässig ist, und wobei die Scheibe (20) zum Innenraum (18) des Gehäuses hin für den Spektralbereich jeder IR-Lichtquelle (10, 12) antireflexbeschichtet ist.

5. Schmierstoffsensor (1) nach einem der vorhergehenden Ansprüche, **dadurch gekennzeichnet, dass** die oder jede IR-Lichtquelle (10, 12) und der oder jeder IR-Detektor (6, 8) auf einer Platine (4) angeordnet sind.

6. Schmierstoffsensor (1) nach Anspruch 5, **dadurch gekennzeichnet, dass** die Platine (4) flexibel ausgebildet ist.

7. Schmierstoffsensor (1) nach einem der vorhergehenden Ansprüche, **dadurch gekennzeichnet, dass** im Gehäuse (2) eine Elektronik (4) eingebracht ist, welche mit dem oder jedem IR-Detektor (10,12) verbunden ist.

8. Wälzlager (40) mit einem Schmierstoffsensor (1) nach einem der vorhergehenden Ansprüche.

## Claims

1. Lubricant sensor (1) comprising a housing (2), an IR light source (10) which is arranged in the housing (2), a first IR detector (6) which is arranged in the housing (2), and a plate (20) which is inserted into an opening in the housing, wherein the plate (20) is transmissive in the spectral range of the IR light source (10), **characterized in that** the plate (20) is provided with an antireflection coating at least for the spectral range of the IR light source (10) in the direction of the interior (18) of the housing, and **in that** a second IR detector (8) is arranged in the housing (2), wherein the first IR detector (6) is arranged in such a way that it is protected against direct irradiation by the IR light source (10), and wherein the second IR detector (8) is arranged in such a way that it is protected against the incident light which is scattered on the lubricant.

2. Lubricant sensor (1) according to Claim 1, **characterized in that** the plate (20) is manufactured from sapphire glass.

3. Lubricant sensor (1) according to Claim 1 or Claim 2, **characterized in that** the IR light source (10) emits light (30, 32) in the near- and/or medium-infrared range.

4. Lubricant sensor (1) according to one of the preceding claims, **characterized in that** a further IR light source (12) is arranged in the housing (2), wherein the plate (20) is transmissive in the spectral range of each IR light source (10, 12), and wherein the plate (20) is provided with an antireflection coating for the spectral range of each IR light source (10, 12) in the direction of the interior (18) of the housing.

5. Lubricant sensor (1) according to one of the preceding claims, **characterized in that** the or each IR light source (10, 12) and the or each IR detector (6, 8) are arranged on a printed circuit board (4).

6. Lubricant sensor (1) according to Claim 5, **characterized in that** the printed circuit board (4) is of flexible design.

7. Lubricant sensor (1) according to one of the preceding claims, **characterized in that** an electronics system (4) which is connected to the or each IR detector (10, 12) is incorporated in the housing (2).

8. Roller bearing (40) having a lubricant sensor (1) according to one of the preceding claims.

## Revendications

1. Capteur de lubrifiant (1) comprenant un boîtier (2), une source de lumière infrarouge (10) disposée dans le boîtier (2), un premier détecteur infrarouge (6) disposé dans le boîtier (2), et un disque (20) inséré dans une ouverture du boîtier, dans lequel le disque (20) est transparent dans le domaine spectral de la source de lumière infrarouge (10), **caractérisé en ce que** le disque (20) est revêtu d'un revêtement antiréfléchissant dans la direction de l'espace intérieur (18) du boîtier pour au moins le domaine spectral de la source de lumière infrarouge (10), et **en ce qu'**un deuxième détecteur infrarouge (8) est disposé dans le boîtier (2), dans lequel le premier détecteur infrarouge (6) est disposé de manière à ce qu'il soit protégé d'une exposition directe à la source de lumière infrarouge (10), et dans lequel le deuxième détecteur infrarouge (8) est disposé de manière à ce qu'il soit protégé de la lumière incidente diffusée par le lubrifiant.

2. Capteur de lubrifiant (1) selon la revendication 1, **caractérisé en ce que** le disque (20) est réalisé en verre saphir.

3. Capteur de lubrifiant (1) selon la revendication 1 ou la revendication 2, **caractérisé en ce que** la source de lumière infrarouge (10) émet une lumière (30, 32) dans le domaine infrarouge proche et/ou moyen.

4. Capteur de lubrifiant (1) selon l'une quelconque des revendications précédentes, **caractérisé en ce qu'**une autre source de lumière infrarouge (12) est disposée dans le boîtier (2), dans lequel le disque (20) est transparent dans le domaine spectral de chaque source de lumière infrarouge (10, 12), et dans lequel le disque (20) est revêtu d'un revêtement antiréfléchissant vers l'espace intérieur (18) du boîtier pour le domaine spectral de chaque source de lumière infrarouge (10, 12).

5. Capteur de lubrifiant (1) selon l'une quelconque des revendications précédentes, **caractérisé en ce que** la ou chaque source de lumière infrarouge (10, 12) et le ou chaque détecteur infrarouge (6, 8) sont disposés sur une carte (4).

6. Capteur de lubrifiant (1) selon la revendication 5, **caractérisé en ce que** la carte (4) est réalisée de manière à être flexible.

7. Capteur de lubrifiant (1) selon l'une quelconque des revendications précédentes, **caractérisé en ce qu'**une électronique (4) est introduite dans le boîtier (2), laquelle électronique est reliée au ou à chaque détecteur infrarouge (10, 12).

8. Palier à roulement (40) pourvu d'un capteur de lubrifiant (1) selon l'une quelconque des revendications précédentes.
